# EUROPEAN PATENT SPECIFICATION

(11) **EP 4 279 048 B1**
(45) Date of publication and mention of the grant of the patent: **23.04.2025**
(21) Application number: 22174298.4
(22) Date of filing: 19.05.2022
(51) Int. Cl.: A61F 13/537

(54) **AN ABSORBENT ARTICLE**
ABSORBIERENDER ARTIKEL
ARTICLE ABSORBANT

(43) Date of publication of application: 22.11.2023
(73) Proprietor: Toiletry Sales Limited, Crigglestone WF4 3HT (GB)
(72) Inventor: SIERRI, Giancarlo, CRIGGLESTONE, WF4 3HT (GB); PATTERSON, Chris, CRIGGLESTONE, WF4 3HT (GB)
(74) Representative: Studio Torta S.p.A.

(56) References cited:
- EP-B1- 0 680 302
- CA-A1- 2 390 959
- KR-B1- 102 184 441
- US-A1- 2006 122 572
- US-A1- 2020 316 245
- US-B1- 10 500 108
- US-B2- 10 772 772
- US-B2- 10 905 604

## Description

### FIELD OF THE INVENTION

The present invention relates to an absorbent article. In particular, the absorbent article comprises layers made of biodegradable and/or bio-sourced materials.

### BACKGROUND OF THE INVENTION

Absorbent articles and particularly disposable absorbent articles of personal hygiene are known in the art. Typical examples include sanitary napkins, panty liners, adult incontinence articles, diapers, paper towels, tampon, interlabial products, perspiration pads, training pants, incontinence underwear, incontinence pull-ons, bed underpad.

Such articles are often used to absorb and retain bodily fluids and other exudates excreted by the human body.

They are usually made in synthetic polymeric material and they are not biodegradable and not compostable. Hence the disposition of the combination of bodily substances and other exudates together with synthetic polymers is particularly polluting.

A disposable absorbent product such as, e.g. a baby diaper, an adult incontinence pad, a sanitary napkin or the like, includes a liquid pervious topsheet, a liquid impervious backsheet, and an absorbent core positioned between the topsheet and the backsheet. The absorbent core has a liquid receiving surface directed towards said topsheet. Preferably an acquisition and distribution layer, also known with the acronym ADL is provided between the topsheet and the absorbent core.

Numerous searches have been done to obtain multilayer materials made of natural and/or biodegradable materials capable of maintaining an adequate acquisition and distribution capacity and an optimal rewetting.

The rewetting is usually measured as the grams of liquid leaving the absorbent core under specified conditions.

The acquisition under pressure is measured as time to distribute a certain amount of bodily fluid.

As many layers are involved in the absorbent article and each layer partially contribute to the different characteristic required to the absorbent article it has been extremely difficult to obtain the desired technical effect associated to the absorbent article with a combination of natural, i.e. bio-sourced and/or biodegradable materials.

Hence, none of the known absorbent articles achieve the desired performance in term of acquisition under pressure and an adequate rewetting capacity and is biodegradable and/or bio-sourced.

US2006122572 discloses a transfer layer of liquid fluids and an absorbent article incorporating the transfer layer, like a diaper, a sanitary napkin or a similar product, which has a permeable cover below which there is disposed a nonwoven transfer layer for liquid fluids followed beneath by an absorbent core which retains the fluids. In the transfer layer there is provided a top layer of predominating hydrophobic properties and a bottom layer of predominating hydrophilic properties. The transfer layer has an embossed surface configuration, with channels formed by compressed nonwoven streaks forming transversal peaks and valleys extending in longitudinal direction along the transfer layer.

CA2390959 discloses disposable absorbent article, e.g., a diaper, and a method of making it. T he chassis is made up of a top-sheet, a fluid acquisition system, and a fluid absorbent core. The fluid acquisition system, comprises a first fluid acquisition layer formed of apertured polymeric, e.g., three dimensional, fi lm and a second fluid acquisition layer, e.g., a fibrous material, secured together and located between the top-sheet and the core, with the first flui d acquisition layer facing the top-sheet and the second fluid acquisition laye r facing the core. The fluid acquisition system serves to facilitate the transference of fluid into the core, e.g., spread out the fluid over the cor e and providing it to the core at a rate at which the materials of the core ca n accommodate. The first and second acquisition layers may be joined or bonded together via various techniques, such as by adhesives, ultrasonic bonding, heat sealing, hot knife-slitting, hydroentanglement, physical stitching or sewing, etc.

### SUMMARY OF THE INVENTION

The aim of the present invention is therefore to provide for an absorbent article comprising a multilayer structure having an excellent acquisition distribution and rewetting capacity and being mainly bio-sourced and/or biodegradable.

According to the present invention the abovementioned aim is achieved by an absorbent article according to claim 1.

### DESCRIPTION OF THE INVENTION

Further characteristics and advantages of the present invention will emerge from the detailed description given below, from the examples provided for illustrative and nonlimiting purposes and from the Figures, in which:

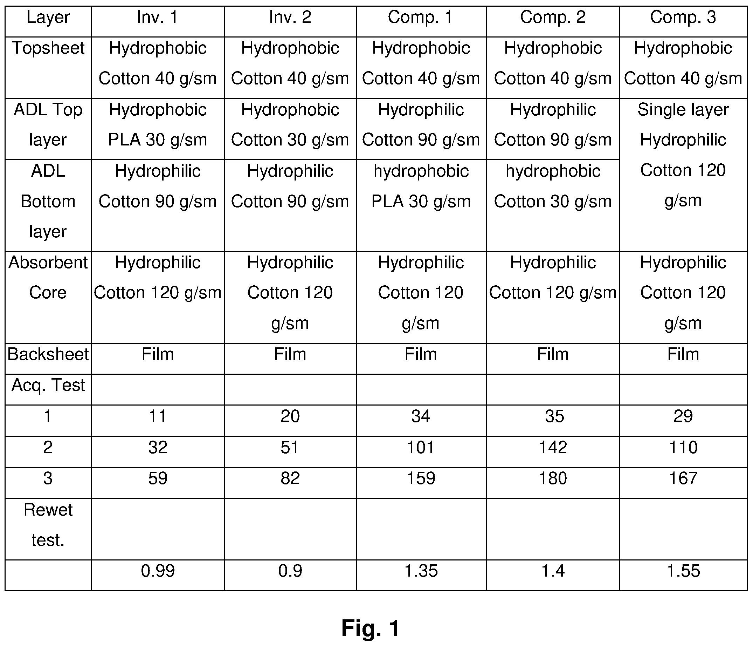

Figure 1 illustrates a table with the composition of inventive and comparative examples of the absorbent article and the results of acquisition and rewet tests.

### DETAILED DESCRIPTION

The term "absorbent article" is used herein in a very broad sense including any article able to receive and/or absorb and/or contain and/or retain fluids and/or exudates, especially bodily fluids/bodily exudates.

Exemplary absorbent articles in the context of the present invention are disposable absorbent articles. The term "disposable" is used herein to describe articles, which are not intended to be laundered or otherwise restored or reused as an article, i.e. they are intended to be discarded after a single use and preferably to be recycled, composted or otherwise disposed of in an environmentally compatible manner. Typical examples include sanitary napkins, panty liners, adult incontinence articles, diapers, interlabial products, perspiration pads, training pants, incontinence underwear, incontinence pull-ons, bed underpad, but also wound dressings. Certain absorbent articles include a fluid pervious topsheet, a fluid impervious backsheet and an absorbent core comprised there between.

The term "use" refers to the period of time that starts when the absorbent article is actually put in contact with the anatomy of a wearer.

By "body fluid" it is meant herein any fluid produced by human body including, but not limited to, perspiration, urine, menstrual fluids, vaginal secretions, faeces and the like.

By "spunlace" is meant a non-woven fabric deriving from a process that consists in the consolidation of the web by means of high pressure water jets that pierce the fabric and intertwine the fibers. Alternative terms for spunlace or spunlaced non-woven fabric are hydro-tangled, hydroenentangled or hydraulic needled. All these terms are included in the present definition of spunlace.

By "biodegradable" is meant a substance capable of being decomposed by bacteria or other living organisms or by natural ecological means avoiding pollution and harmful bi product materials being retained by the natural environment.

By "bio-sourced" or "bio-based" is meant a material intentionally made from substances derived from living or once-living organisms, in other terms all materials made from raw materials derived from biological or renewable resources, such as cotton, bamboo, cellulose, PLA, but also bio-PE and bio-PET. Bio-based plastic also offers many benefits. In most cases, bio-based plastic has the same attributes as fossil-based plastic, but with a significantly better eco-balance.

By "layer" is meant a mass of homogeneous material spread out more or less uniformly on a surface. A layer may vary in thickness, but the material remain homogenous in all the mass.

The term "Nonwoven" means engineered fibrous assembly, primarily planar, which has been given a designed level of structural integrity by physical and/or chemical means, excluding weaving, knitting or papermaking (ISO 9092:2019 2019).

The term "superabsorbent" polymers or SAP refers to polymers absorbing and retaining extremely large amounts of a liquid relative to their own mass. Such water-absorbing polymers, that are classified as hydrogels when crosslinked, absorb aqueous solutions through hydrogen bonding with water molecules. A SAP's ability to absorb water depends on the ionic concentration of the aqueous solution. SAP materials used in manufacturing diapers are characterized of having a free absorbency to distillate water higher than 100 g/g. Such materials include the preferred biodegradable and/or biosourced SAP and the particularly preferred polymers described in WO2019195272 and/or WO2014125418.

The term "airlaid" means a web of fibres produced by airlaying. The term "airlaying" means forming a web by dispersing fibres in an air stream and condensing them from the air stream onto a moving screen by means of a pressure or vacuum. The term "airlaid nonwoven" means an airlaid web bonded by one or more techniques to provide fabric integrity.

According to an embodiment the absorbent article comprises a multilayer structure comprising at least a topsheet, an acquisition and distribution layer also known as ADL, an absorbent core and a back sheet, but it may comprise further layers.

Topsheet, ADL, absorbent core and back-sheet are all "layers" under the above definition.

With the terms "Hydrophobic layer" and "hydrophilic layer" are meant layers comprising at least 80% of hydrophobic fibres and hydrophilic fibres respectively in order to be actually Hydrophobic and hydrophilic.

The topsheet comprises and preferably is made of biodegradable and/or bio-sourced hydrophobic fibres, more preferably of hydrophobic fibres chosen in the group constituted by cotton, bamboo, PLA, viscose and their mixtures and comprises holes in order to allow the absorption of fluid, but having dimensions capable to avoid the rewetting of the surface in contact with the body of the user.

The topsheet is preferably a nonwoven or a spunlace.

The weight of the topsheet is preferably between 15 and 50 g/sm, i.e. gram per square meter, more preferably between 30 and 40 g/sm.

The open area of the topsheet is preferably between 1 and 50%, more preferably between 1 and 35%, even more preferably between 10 and 20 %.

The perforation or holes patterns my greatly differ.

The holes are approximately circular, but in view of the different perforating methods, it may also be elliptical, particularly in the lighter topsheets, or may also have different shape as polygonal shapes or regular and the shape generally depend on the perforation method.

It is also important to note that in real absorbent articles the shape of the holes may only resemble a circumference or an ellipse and the shape may look irregular.

The average diameter of the holes is preferably between 0.5 and 4 mm, more preferably between 1 and 2 mm, even more preferably between 1.2 and 1.8 mm.

By "diameter" in case of the elliptical shape of the hole is intended the minor axis of the ellipse and in case of other shapes is the height of the hole.

The acquisition distribution layer or ADL may also be called top core and is preferably a laminated, coupled or composite double layer having an average weight per square meter preferably comprised between 40 and 200 g/sm, more preferably between 80 and 160 g/sm, as an example 120 g/sm.

The double layer is perforated with through holes and comprises a first top layer, directed towards the topsheet or the user when in use and a bottom layer directed towards the absorbent core. The two layers are in contact with each other.

The ADL double layer is perforated in a single step to allow the holes to correctly distribute the fluid.

The holes of the acquisition distribution layer are approximately circular, but in view of the different perforating methods, it may also be elliptical or have different shape.

The diameter of the holes is preferably between 0.5 and 4 mm, more preferably between 0.5 and 1.5 mm.

Preferably, the first or top layer is a spunlace layer.

Preferably the top layer has a weight comprised between 20 and 60 g/sm, more preferably between 30 and 40 g/sm.

Preferably the top layer is a hydrophobic layer. Preferably the hydrophobic fibres are biodegradable fibres and/or bio-sourced fibres.

Preferably the hydrophobic biodegradable fibres and/or bio-sourced fibres are chosen in the group constituted by hydrophobic cotton, hydrophobic bamboo, hydrophobic PLA, hydrophobic viscose fiber and their mixtures.

The second or bottom layer of the double layer forming the acquisition distribution layer is preferably made of hydrophilic biodegradable and/or bio-sourced fibres.

Preferably the bottom layer is also a spunlace layer.

Preferably the bottom layer has a weight preferably comprised between 20 and 180 g/sm, more preferably between 60 and 100 g/sm, for example 90 g/sm.

Preferably the hydrophilic biodegradable fibres and/or bio-sourced fibres are chosen in the group constituted by hydrophilic cotton, hydrophilic bamboo, hydrophilic viscose, hydrophilic cellulose fiber.

Both the layers of the double layer are perforated and comprise through holes. The double layer may be perforated with known techniques.

The open area of the acquisition and distribution layer is preferably between 1 and 50%, more preferably between 5 and 35 %, even more preferably between 10 and 20 %, as an example 19%.

The perforation or holes patterns my greatly differ.

It has been surprisingly verified that the spunlace layers are the only layers capable of assuring an excellent acquisition distribution and rewetting capacity, when associated to biodegradable and/or bio-sourced material.

Moreover the succession of hydrophilic materials and hydrophobic materials is an essential feature that allows for improved results in term of an acquisition and distribution and rewetting capacity.

The absorbent core or lower core is usually directly in contact with the acquisition distribution layer and is preferably made of hydrophilic fibres, more preferably of hydrophilic fibres selected from the group constituted by hydrophilic cotton, hydrophilic bamboo, hydrophilic viscose, hydrophilic cellulose fiber and/or a superabsorbent. Among the superabsorbents are preferred superabsorbents biodegradable or biosourced, at least partially.

The absorbent core is preferably a spunlace or airlaid layer, more preferably a spunlace.

The absorbent article further comprises a back sheet. Preferably the back sheet is of biodegradable and/or bio-sourced material. Preferably the back-sheet is a spunlace of biodegradable and/or bio-sourced material. The biodegradable and/or bio-sourced material includes preferably hydrophobic fibres chosen in the group constituted by cotton or bamboo and their mixtures. Alternatively the back-sheet is a film of biodegradable and/or bio-sourced material, preferably the biodegradable and/or bio-sourced material is chosen in the group constituted by PLA or MATER-BI and their mixtures.

The Invention will be described now by way of examples, but is not limited to these examples.

### EXAMPLES 1-2 AND COMPARATIVE EXAMPLES 1-3

Exemplary absorbent articles with particularly preferred layers according to the invention are given in Figure 1 and compared with comparative absorbent articles. In particular, two inventive examples 1 and 2 using different materials in the ADL are compared with comparative structure 1 and 2 wherein the ADL top layer and ADL bottom layer are inverted and therefore the succession of the layers constituting the ADL is the opposite.

Comparative example 3 refers to a known structure wherein the ADL is constituted by a single layer of hydrophilic cotton.

An acquisition test and a rewetting test are made according to the procedure explained below.

A Synthetic Menstrual Fluid (SMF) is prepared comprising: Tylose H 20 P 2 (hydroxyethyl cellulose), NaCl, NaHCO3, color Methylene Blue, glycerine 86%, distilled water. In 800 ml of water 14 g of Tylose are introduced, under agitation in 20 min. Then 10 g of NaCl are added under agitation in 5 min, and subsequently 4 g of NaHCO3, 2 g of color, and 100 g of glycerine. Then distilled water as needed up to 1 liter.

### Rewet test

The rewet test method is used to assess the dryness of the absorbent structure with respect to its wearer facing surface. The test fluid that is utilized for this test is the Synthetic Menstrual Fluid (SMF) prepared as above.

Apparatus needed:
1) Blotting Paper available from Whatman (Germany) S & S Rundfilter / Durchmesser 150 mm, No.; 597, Reference-No.: 311812.
2) A weight of 3500 g covered on the lower surface with a foam of moderate flexibility. Both the weight and foam are covered with a thin, flexible plastic film to avoid the foam absorbing fluid. The weight dimensions should allow a 5 cm x 10 cm surface to contact the sample under examination. Pressure exerted onto the sample = 70 g/cm2 .
3) A perspex (7 mm thick) plate of dimensions 6 cm x 10 cm with a hole of dimensions 3 cm x 4 cm centered in the template.
4) A burette capable of introducing the test fluid at a reproducible rate of 7 ml in 90 seconds.
5) An analytical balance capable of reading to 4 decimal places.

### Sample preparation/measurement:

The sample to be tested is an absorbent feminine pad with wings.

A 285 mm x 100/155 mm sample with wings to be assessed is placed on a flat laboratory surface with the cover layer facing up and centered directly below the burette for test liquid delivery. The perspex plate is positioned on the sample, centered on it, and the SMF test liquid is introduced over the exposed area corresponding to the hole in the perspex plate.

After 90 seconds 7 ml of SMF have been introduced to the sample and an electronic counter set to 20 min is activated. During this waiting period a stack of 6 discs of filter paper are weighed on the analytical balance and the weight is recorded.

After 20 min the perspex plate is removed and the stack of filter papers is positioned centrally on the sample being assessed and the weight is gently lowered onto the filter paper stack. The sample and filter paper stack remain under the pressure exerted by the weight for a period of 15 seconds, after which the weight is carefully removed and the filter paper stack is re-weighed. The difference in weight is recorded as the Rewet value. Each test is repeated for at least 5 samples and averaged to ensure adequate accuracy of the measurements.

### Acquisition Test

This acquisition method measures a product's ability to keep an absorbing capacity to repeated assaults of fluid under a prescribed set of conditions. This method evaluates the time required for the acquisition of given amounts of liquid during repeated imbibitions (three imbibitions of 4 ml each), at relatively high speed (about 3 ml/sec) and under pressure of 1723.7 Pa (0.25 PSI), to model in use pressure while wearing the product.

Each sample with wings has dimensions of 285 mm x 100/155 mm and is laid down on a flat surface with the topsheet layer facing up. An acquisition plate is placed on the sample and centered on the sample. The acquisition plate comprises a rectangular plexiglas plate 65 mm x 180 mm x 8 mm with an aperture 25.4 mm in diameter formed in the centre therein. A cylinder 45 mm high and 22 mm in internal diameter is located over the aperture in sealing contact with the plate. The cylinder is filled with Synthetic Menstrual Fluid (SMF), and a pressure of 1723.7 Pa (0.25 PSI) is applied, obtained with appropriate weights positioned on the plate, the pressure being that measured with reference to the portion of the sample under the acquisition plate. The acquisition time is the time from the beginning of each imbibition until the disappearance of the liquid from the interior of the cylinder. A waiting time of 5 minutes is left after each imbibition before repeating the procedure.

The 3 acquisition times are recorded as the Acquisition of the absorbent structure. Each test is repeated for at least 5 samples and averaged to ensure adequate accuracy of the measurements.

The compositions and the result of the tests are drawn in table 1 of Fig. 1.

The inventive combination of spunlace layers and the selection of materials outperforms the comparative absorbent articles.

As it can be noted the inversion of the two layers constituting the ADL in the comparative examples 1 and 2 worsen the results in both tests and it is therefore clear that the succession of hydrophilic and hydrophobic layers in a double layer ADL is critical for obtaining the best results.

## Claims

1. An absorbent article comprising at least a topsheet, an acquisition and distribution layer and an absorbent core, **characterized in that**
a. said topsheet is made of hydrophobic fibres
b. said acquisition and distribution layer is perforated with through holes and comprises:
i. a top spunlace layer, made of hydrophobic fibres selected in the group constituted by cotton, bamboo, PLA, viscose and their mixtures
ii. a bottom spunlace layer made of hydrophilic fibres selected in the group constituted by cotton, bamboo, viscose, cellulose and their mixtures
c. said absorbent core is made of hydrophilic fibres.

2. The absorbent article according to claim 1, **characterized in that** said topsheet is made of hydrophobic fibres selected in the group constituted by cotton, bamboo, PLA, viscose and their mixtures.

3. The absorbent article according to claims 1 or 2, **characterized in that** said top spunlace layer is made of bamboo or cotton.

4. The absorbent article according to any of claims 1 to 3, **characterized in that** said bottom spunlace layer is made of bamboo or cotton.

5. The absorbent article according to any of the preceding claims, **characterized in that** said absorbent core comprises a spunlace or airlaid material made of hydrophilic fibres.

6. The absorbent article according to any of the preceding claims, **characterized in that** said absorbent core comprises at least a material selected in the group constituted by cotton, bamboo, viscose, cellulose, superabsorbent polymers and their mixtures.

7. The absorbent article according to any of the preceding claims, **characterized in that** said absorbent core comprises at least a material selected in the group consisting of cellulose, cotton, a superabsorbent polymer and their mixtures.

8. The absorbent article according to claim 7, **characterized in that** it further comprises a back sheet made of biodegradable and/or bio-sourced materials.

9. The absorbent article according to any of the preceding claims, **characterized in that** said top spunlace layer has a weight comprised between 20 and 60 g/sqm.

10. The absorbent article according to any of the preceding claims, **characterized in that** the weight of the bottom spunlace layer is between 20 and 180 g/sqm.

11. The absorbent article according to any of the preceding claims, **characterized in that** said acquisition and distribution layer has an open area of 5 to 35 %.

12. The absorbent article according to any of the preceding claims, **characterized in that** the dimension of the diameter of the holes of the acquisition and distribution layer is between 1 and 2 mm.

## Patentansprüche

1. Absorberartikel umfassend mindestens eine Oberschicht, eine Aufnahme- und Verteilungsschicht und einen Absorberkern, **dadurch gekennzeichnet, dass**
a. die Oberschicht aus hydrophoben Fasern besteht
b. die Aufnahme- und Verteilungsschicht ist mit Durchgangsbohrungen perforiert und umfasst:
i. eine Spunlace Vliesstoff-Oberschicht, die aus hydrophoben Fasern besteht, die aus der Gruppe bestehend aus Baumwolle, Bambus, PLA, Viskose und deren Mischungen ausgewählt werden
ii. eine Spunlace Vliesstoff Unterschicht, die aus hydrophilen Fasern besteht, die aus der Gruppe bestehend aus Baumwolle, Bambus, Viskose, Zellulose und deren Mischungen ausgewählt werden
c. der Absorberkern aus hydrophilen Fasern besteht.

2. Absorberartikel nach Anspruch 1, **dadurch gekennzeichnet, dass** die Oberschicht aus hydrophoben Fasern besteht, die aus der Gruppe bestehend aus Baumwolle, Bambus, PLA, Viskose und deren Mischungen ausgewählt werden.

3. Absorberartikel nach Anspruch 1 oder 2, **dadurch gekennzeichnet, dass** die Spunlace Vliesstoff Oberschicht aus Bambus oder Baumwolle besteht.

4. Absorberartikel nach einem der Ansprüche 1 bis 3, **dadurch gekennzeichnet, dass** die Spunlace Vliesstoff Unterschicht aus Bambus oder Baumwolle besteht.

5. Absorberartikel nach einem der vorhergehenden Ansprüche, **dadurch gekennzeichnet, dass** der Absorberkern ein Spunlace Vliesstoff oder Airlaid-Material umfasst, das aus hydrophilen Fasern besteht.

6. Absorberartikel nach einem der vorhergehenden Ansprüche, **dadurch gekennzeichnet, dass** der Absorberkern mindestens ein Material umfasst, das aus der Gruppe bestehend aus Baumwolle, Bambus, Viskose, Zellulose, superabsorbierenden Polymeren und deren Mischungen ausgewählt wird.

7. Absorberartikel nach einem der vorhergehenden Ansprüche, **dadurch gekennzeichnet, dass** der Absorberkern mindestens ein Material umfasst, das aus der Gruppe bestehend aus Zellulose, Baumwolle, einem superabsorbierenden Polymer und deren Mischungen ausgewählt wird.

8. Absorberartikel nach Anspruch 7, **dadurch gekennzeichnet, dass** er ferner eine Rückschicht umfasst, die aus biologisch abbaubaren Materialen und/oder aus Materialen aus biologischer Ursprung besteht.

9. Absorberartikel nach einem der vorhergehenden Ansprüche, **dadurch gekennzeichnet, dass** die Spunlace Vliesstoff Oberschicht ein Gewicht zwischen 20 und 60 g/qm aufweist.

10. Absorberartikel nach einem der vorhergehenden Ansprüche, **dadurch gekennzeichnet, dass** das Gewicht der Spunlace Vliesstoff Unterschicht zwischen 20 und 180 g/qm liegt.

11. Absorberartikel nach einem der vorhergehenden Ansprüche, **dadurch gekennzeichnet, dass** die Aufnahme- und Verteilungsschicht eine offene Fläche von 5 bis 35 % aufweist.

12. Absorberartikel nach einem der vorhergehenden Ansprüche, **dadurch gekennzeichnet, dass** die Größe des Durchmessers der Bohrungen der Aufnahme- und Verteilungsschicht zwischen 1 und 2 mm liegt.

## Revendications

1. Article absorbant comprenant au moins une feuille supérieure, une couche d'acquisition et de répartition et un noyau absorbant, **caractérisé par le fait que**
a. ladite feuille supérieure est constituée de fibres hydrophobes
b. ladite couche d'acquisition et de répartition est perforée de trous traversants et comprend :
i. une couche supérieure de spunlace, constituée de fibres hydrophobes choisies dans le groupe constitué par le coton, le bambou, l'APL, la viscose et leurs mélanges
ii. une couche inférieure de spunlace constituée de fibres hydrophiles choisies dans le groupe constitué par le coton, le bambou, la viscose, la cellulose et leurs mélanges
c. ledit noyau absorbant est constitué de fibres hydrophiles.

2. Article absorbant selon la revendication 1, **caractérisé par le fait que** ladite feuille supérieure est constituée de fibres hydrophobes choisies dans le groupe constitué par le coton, le bambou, l'APL, la viscose et leurs mélanges.

3. Article absorbant selon les revendications 1 ou 2, **caractérisé par le fait que** ladite couche supérieure de spunlace est en bambou ou en coton.

4. Article absorbant selon l'une quelconque des revendications 1 à 3, **caractérisé par le fait que** ladite couche inférieure de spunlace est en bambou ou en coton.

5. Article absorbant selon l'une quelconque des revendications précédentes, **caractérisé par le fait que** ledit noyau absorbant comprend une matière spunlace ou airlaid constituée de fibres hydrophiles.

6. Article absorbant selon l'une quelconque des revendications précédentes, **caractérisé par le fait que** ledit noyau absorbant comprend au moins une matière choisie dans le groupe constitué par le coton, le bambou, la viscose, la cellulose, les polymères superabsorbants et leurs mélanges.

7. Article absorbant selon l'une quelconque des revendications précédentes, **caractérisé par le fait que** ledit noyau absorbant comprend au moins une matière choisie dans le groupe constitué par la cellulose, le coton, un polymère superabsorbant et leurs mélanges.

8. Article absorbant selon la revendication 7, **caractérisé par le fait qu'**il comprend en outre une feuille arrière constituée de matières biodégradables et/ou biosourcées.

9. Article absorbant selon l'une quelconque des revendications précédentes, **caractérisé par le fait que** ladite couche supérieure de spunlace a un poids compris entre 20 et 60 g/m².

10. Article absorbant selon l'une quelconque des revendications précédentes, **caractérisé par le fait que** le poids de la couche inférieure de spunlace est compris entre 20 et 180 g/m².

11. Article absorbant selon l'une quelconque des revendications précédentes, **caractérisé par le fait que** ladite couche d'acquisition et de répartition présente une surface ouverte de 5 à 35 %.

12. Article absorbant selon l'une quelconque des revendications précédentes, **caractérisé par le fait que** la dimension du diamètre des trous de la couche d'acquisition et de répartition est comprise entre 1 et 2 mm.
